# EUROPÄISCHE PATENTSCHRIFT

(11) **EP 1 097 679 B1**
(45) Veröffentlichungstag und Bekanntmachung des Hinweises auf die Patenterteilung: **19.05.2004**
(21) Anmeldenummer: 00810904.3
(22) Anmeldetag: 02.10.2000
(51) Int. Cl.: A61F 2/38

(54) **Monokondyläre Kniegelenkprothese**
Unicompartmental knee prosthesis
Prothèse du genou a un seul compartiment

(30) Priorität: 29.10.1999 EP 99810980
(43) Veröffentlichungstag der Anmeldung: 09.05.2001
(73) Patentinhaber: Sulzer Orthopädie AG, 6340 Baar (CH)
(72) Erfinder: Aicher, Martin, 78601 Mahlstetten (DE); Leclercq, Vincent, 8404 Winterthur (CH); Gyssler, Bernhard, 8810 Horgen (CH)
(74) Vertreter: Manitz, Finsterwald & Partner Gbr

(56) Entgegenhaltungen:
- WO-A-95/27450
- DE-C- 2 660 623
- GB-A- 2 312 377
- US-A- 4 085 466
- US-A- 4 353 136

## Beschreibung

Die Erfindung betrifft eine monokondyläre Kniegelenkprothese gemäss dem Oberbegriff des unabhängigen Patentanspruchs.

Monokondyläre Kniegelenkprothesen kommen dort zum Einsatz, wo nicht die gesamte Gelenkverbindung des Kniegelenks, also sowohl die mediale als auch die laterale Gelenkverbindung, nicht mehr intakt ist, sondern nur eine Hälfte (entweder die mediale oder die laterale Hälfte), wo aber die Bänder (Seitenbänder und Kreuzbänder) noch vollständig intakt sind. In diesen Fällen braucht keine bikondyläre Prothese implantiert zu werden. Auf diese Weise kann die vorhandene Knochensubstanz so weit wie möglich geschont werden und auch die Bänder können so weit wie möglich erhalten werden.

Eine solche monokondyläre Kniegelenkprothese umfasst typischerweise ein Femurteil, welches eine konvexe Lauffläche aufweist, und welches entweder zementfrei oder zementiert an dem zuvor einseitig präparierten Femur fixiert wird. Femer umfasst eine derartige Kniegelenkprothese ein Tibiateil, welches entsprechend an der zuvor einseitig präparierten Tibia fixiert wird. Zwischen dem am Femur fixierten Femurteil, welches typischerweise metallisch ausgebildet ist, und dem an der Tibia fixierten Tibiateil, welches ebenfalls typischerweise metallisch ausgebildet ist, ist typischerweise noch ein Zwischenteil angeordnet, das typischerweise aus Kunststoff (z.B. aus ultrahochmolekularem Polyethylen) hergestellt ist und entweder gleitend verschiebbar auf einer Gleitfläche des Tibiateils angeordnet ist oder relativ zu dem Tibiateil unverschiebbar, also fest, angeordnet ist.

Bei einer bekannten monokondylären Kniegelenkprothese, wie sie z.B. in der US-A-4,085,466 beschrieben ist, ist das Zwischenteil im wesentlichen frei verschiebbar auf der Gleitfläche des Tibiateils angeordnet. Bei einer solchen Art von Kniegelenkprothese muss die gesamte Führung der Bewegung vom Muskel- und Bandapparat übernommen werden. Die Begrenzung der Beweglichkeit des Zwischenteils erfolgt nur durch die Muskeln und Bänder bzw. durch die Weichteile. Da keine seitliche Begrenzung der Beweglichkeit des Zwischenteils vorhanden ist, müssen Kräfte in medialer/lateraler Richtung, die zuvor teilweise von den Menisci bzw. vom Knochenlager aufgenommen wurden, ebenfalls von den Muskel und Bändern aufgenommen werden bzw. von dem noch intakten Knochenlager.

Bei einem anderen Ausführungsbeispiel der US-A-4,085,466 kann eine Begrenzungsfläche vorgesehen sein, welche die Gleitfläche seitlich begrenzt. So kann z.B. bei einem Tibiateil der lateralen Hälfte der Gelenkverbindung im Bereich der medialen Begrenzung des Tibiateils (also im Bereich der eminentia intercondylaris) eine seitliche Begrenzungsfläche am Tibiateil vorgesehen sein, welche die Gleitfläche des Tibiateils seitlich begrenzt. Diese seitliche Begrenzungsfläche verläuft geradlinig in anteriorer/posteriorer Richtung und dient als Anschlagfläche für diejenige Seitenfläche des Zwischenteils, welche der Begrenzungsfläche des Tibiateils zugewandt ist bzw. sie dient als Führungsfläche, entlang welcher das Zwischenteil geführt wird. Diese Anschlagfläche kann also sowohl Kräfte in medialer(bzw. bei einem Ersatz der medialen Hälfte in lateraler) Richtung aufnehmen und auch das Zwischenteil in anteriorer/posteriorer Richtung führen.

Die Kinematik des natürlichen Kniegelenks soll nach Möglichkeit durch eine monokondyläre Kniegelenkprothese nicht beeinflusst oder verändert werden. Bei der Implantation einer monokondylären Kniegelenkprothese wird der Gelenkknorpel und der Meniskus ersetzt (durch Tibiateil und Femurteil bzw. durch das Zwischenteil), die Verbindung zwischen dem Meniskus und dem Knochen, die beim natürlichen Kniegelenk vorhanden ist, ist aber nach der Implantation nicht vorhanden, weil bei der monokondylären Kniegelenkprothese das Zwischenteil ohne eine Verbindung zum Tibiateil beweglich ist. Für das Zwischenteil wird daher eine Führung benötigt, um der physiologischen Versetzung der Kondylen zu folgen.

Nach neueren Erkenntnissen über die Kinematik des menschlichen Kniegelenks ist es allerdings so, dass der femoro-tibiale Kontaktpunkt eine Bahn beschreibt, welche durch eine geradlinige Bahn - wie sie weiter oben beschrieben ist - nicht besonders gut approximiert werden kann. Das heisst, wenn man die tatsächliche Bahn des femoro-tibialen Kontaktpunkts durch eine geradlinige Bahn approximiert, wie dies bei der oben beschriebenen Führung entlang der geradlinig in anteriorer/posteriorer Richtung verlaufenden Begrenzungsfläche erfolgt, dann wird der Bandapparat stärker belastet als dies beim natürlichen, intakten Knie der Fall ist.

Hier will die Erfindung Abhilfe schaffen. Es ist daher eine Aufgabe der Erfindung, eine monokondyläre Kniegelenkprothese vorzuschlagen, welche die vorstehend beschriebenen Nachteile nicht aufweist. Die Prothese soll einerseits eine geführte Bewegung herbeiführen, welche der natürlichen Bewegung des Knies so gut wie möglich entspricht, und die Muskeln und Bänder sollen so natürlich beansprucht werden wie möglich. Andererseits soll die Prothese auch in der Lage sein, Kräfte in medialer/lateraler Richtung aufzunehmen, sodass solche Kräfte von der Prothese und dann letztlich vom Knochen, also von der Tibia, aufgenommen werden können und nicht zusätzlich von den Muskeln und Bänder bzw. von der intakten Hälfte des Knochenlagers aufgenommen werden müssen.

Erfindungsgemäss wird daher eine monokondyläre Kniegelenkprothese vorgeschlagen wie sie durch die Merkmale des unabhängigen Patentanspruchs charakterisiert ist. Besonders vorteilhafte Ausgestaltungen der erfindungsgemässen monokondylären Kniegelenkprothese ergeben sich aus den Merkmalen der abhängigen Patentansprüche.

Die erfindungsgemässe monokondyläre Kniegelenkprothese umfasst also ein Femurteil, welches eine konvexe Lauffläche aufweist. Ferner umfasst sie ein Tibiateil mit einer dem Femurteil zugewandten Gleitfläche und ein Zwischenteil, welches bei der Implantation zwischen das Femurteil und das Tibiateil eingebracht wird. Das Zwischenteil weist zwei Lagerflächen auf, nämlich eine erste, konkave Lagerfläche zum Zusammenwirken mit der konvexen Lauffläche des Femurteils, sowie eine zweite Lagerfläche zum Zusammenwirken mit der Gleitfläche des Tibiateils. Am Tibiateil ist eine seitliche Begrenzungsfläche vorgesehen, welche die Gleitfläche des Tibiateils seitlich begrenzt und in Richtung auf das Femurteil weisend vorsteht, um mit einer Seitenfläche des Zwischenteils zusammenwirken zu können. Die am Tibiateil vorgesehene seitliche Begrenzungsfläche ist bogenförmig gekrümmt ausgebildet und das Zwischenteil weist eine Seitenfläche auf, welche entsprechend der am Tibiateil vorgesehenen, bogenförmig gekrümmten seitlichen Begrenzungsfläche gekrümmt ist.

Bei einem bevorzugten Ausführungsbeispiel ist die am Tibiateil vorgesehene seitliche Begrenzungsfläche kreissegmentförmig gekrümmt ausgebildet, und die Seitenfläche des Zwischenteils ist entsprechend kreissegmentförmig gekrümmt ausgebildet.

Die bogenförmige, insbesondere kreissegmentförmig gekrümmte Begrenzungsfläche und die entsprechend dieser Begrenzungsfläche gekrümmte Seitenfläche des Zwischenteils stellen eine sehr gute Approximation der natürlichen Bahn des femoro-tibialen Kontaktpunktes dar, wodurch die Muskeln und Bänder ähnlich belastet werden wie beim natürlichen, intakten Knie. Ausserdem können in lateraler/medialer Richtung wirkende Kräfte von der Prothese bzw. letztlich von der Tibia aufgenommen werden und müssen nicht wie bei einer Prothese mit frei beweglichem Zwischenteil vom Muskel- bzw. Bandapparat aufgenommen werden.

Bei einem weiteren vorteilhaften Ausführungsbeispiel der erfindungsgemässen monokondylären Kniegelenkprothese ist die am Tibiateil vorgesehene seitliche Begrenzungsfläche konvex und die entsprechende Seitenfläche des Zwischenteils konkav ausgebildet. Dies ist deshalb von Vorteil, weil die laterale Kondyle bei einer Flexions-/Extensionsbewegung den deutlich grösseren Weg zurücklegt als die mediale Kondyle, und die Bahn des femoro-tibialen Kontaktpunkts der lateralen Kondyle besonders gut durch eine Bewegung entlang einer konvexen, insbesondere kreissegmentförmig konvexen Bahn verläuft.

Bei einem weiteren vorteilhaften Ausführungsbeispiel liegt der Krümmungsradius der seitlichen Begrenzungsfläche des Tibiateils und dementsprechend der Krümmungsradius der Seitenfläche des Zwischenteils im Bereich von 30-50 mm. Dieser Bereich umfasst den wesentlichen Bereich der Radien, mit denen eine Bewegung des femoro-tibialen Kontaktpunkts approximiert wird, wie sie beim natürlichen Knie vorkommt.

Bei einem weiteren vorteilhaften Ausführungsbeispiel liegt die Breite des Tibiateils im Bereich von 15-55 mm und die Länge des Tibiateils liegt im Bereich von 35-60 mm. Diese Bereiche für die Abmessung von Breite und Länge des Tibiateils überdecken die am häufigsten beim Menschen vorkommenden Abmessungen der Tibia.

Schliesslich erstreckt sich bei einem weiteren vorteilhaften Ausführungsbeispiel die bogenförmige Seitenfläche des Zwischenteils über einen Bogenwinkel, der im Bereich von 25°-45° liegt. Damit ist gewährleistet, dass das Meniskusteil eine ausreichende Grösse aufweist, um eine ausreichend grosse Kontaktfläche zwischen der Lauffläche des Femurteils und der Lagerfläche des Zwischenteils zu gewährleisten.

Im folgenden wird die Erfindung anhand der Zeichnung näher erläutert. Dabei zeigen in schematischer Darstellung:
- Fig. 1: eine Aufsicht auf ein Ausführungsbeispiel eines Tibiateils einer erfindungsgemässen monokondylären Kniegelenkprothese,
- Fig. 2: das Ausführungsbeispiel des Tibiateils aus Fig. 1 in Seitenansicht,
- Fig. 3: das Ausführungsbeispiel des Tibiateils aus Fig. 1 in einer Ansicht von unten,
- Fig. 4: eine perspektivische Ansicht eines Zwischenteils einer erfindungsgemässen monokondylären Kniegelenkprothese,
- Fig. 5: eine Aufsicht auf das Zwischenteil aus Fig. 4,
- Fig. 6: eine perspektivische Ansicht eines Femurteils einer erfindungsgemässen monokondylären Kniegelenkprothese,
- Fig. 7: eine Seitenansicht des Femurteils aus Fig. 6.

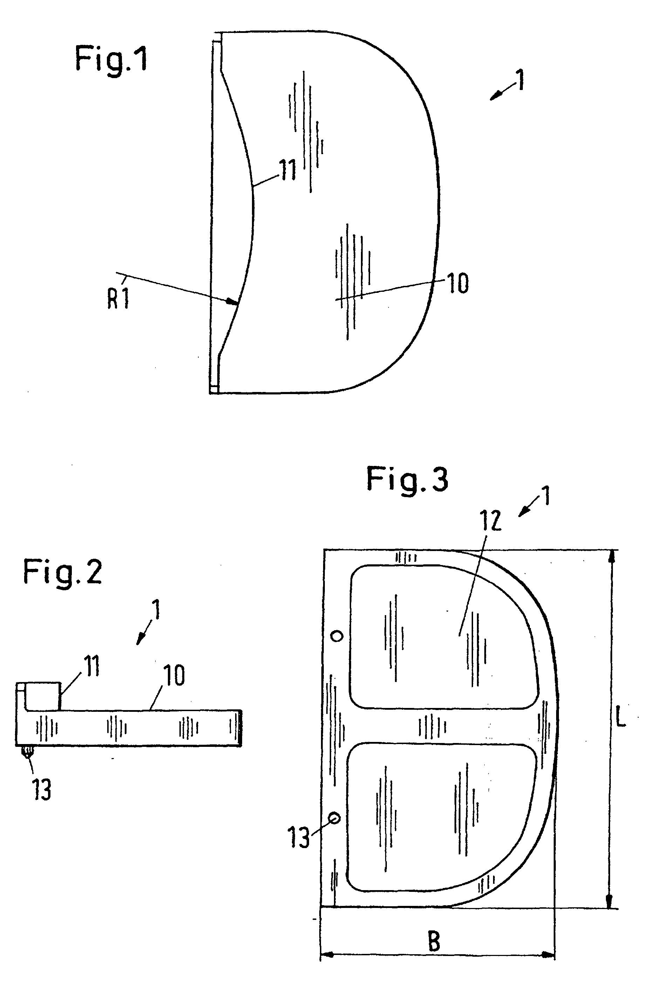

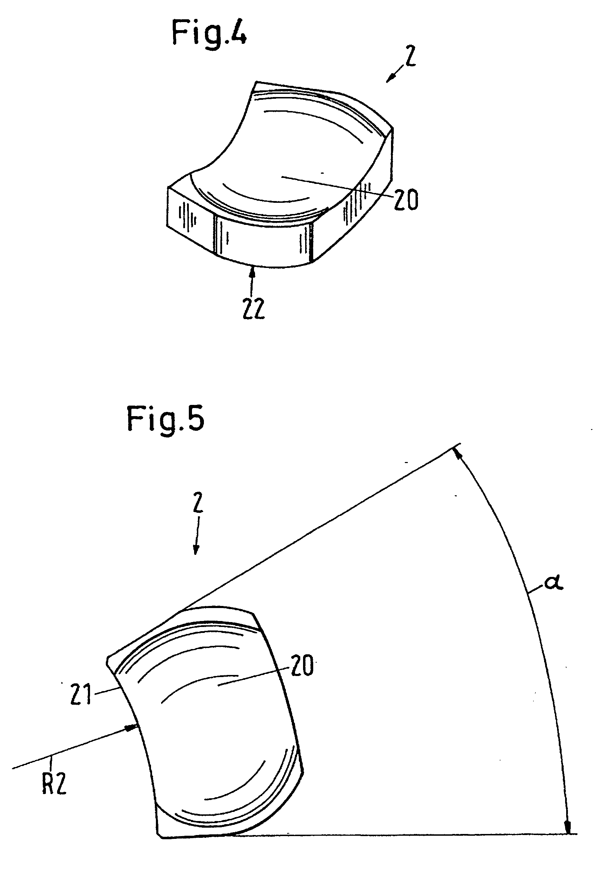

In Fig. 1-3 ist ein Ausführungsbeispiel eines Tibiateils einer erfindungsgemässen monokondylären Kniegelenkprothese dargestellt. Das Tibiateil 1 umfasst eine Gleitfläche 10, auf welcher ein Zwischenteil 2 (siehe Fig. 4 bzw. Fig. 5) gleitend verschiebbar ist. Am Tibiateil 1 ist eine seitliche Begrenzungswand mit einer seitlichen Begrenzungsfläche 11 vorgesehen, welche die Gleitfläche 10 seitlich begrenzt. Die Begrenzungsfläche 11 ist .bogenförmig, insbesondere kreissegmentförmig, gekrümmt und ist bei dem beschriebenen Ausführungsbeispiel konvex ausgebildet. Der Krümmungsradius dieser Begrenzungsfläche 11 ist mit R1 bezeichnet.

Auf seiner Unterseite weist das Tibiateil Zementtaschen 12 (siehe Fig. 3) auf für eine zementierte Befestigung des Tibiateils 1 an der Tibia. Vom Prinzip her kann das Tibiateil aber auch so ausgebildet sein, dass es Mittel für eine zementfreie Verankerung in der Tibia aufweist. Ferner erkennt man in Fig. 2, dass von der Unterseite zwei kleine Fixationszapfen 13 abstehen, welche bei einer Implantation des Tibiateils 1 ein Verrutschen des Tibiateils 1 auf der Tibia verhindern, solange der Knochenzement noch nicht ausreichend ausgehärtet ist.

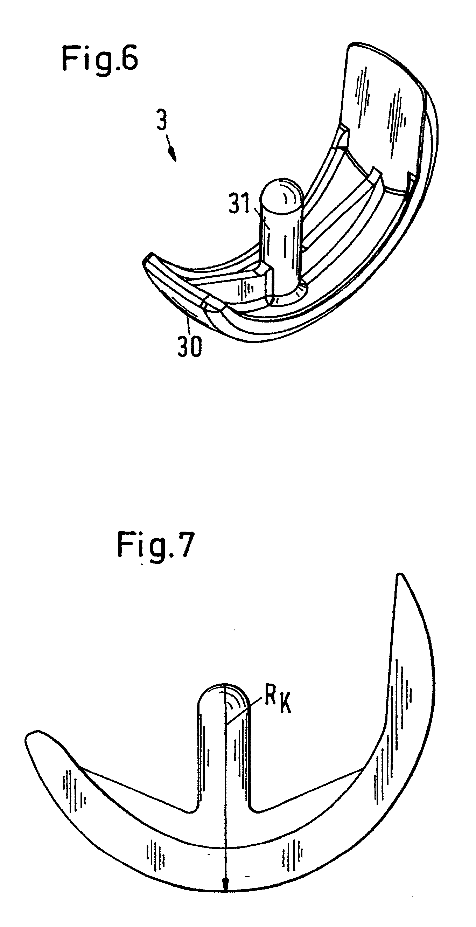

In Fig. 4 und Fig. 5 ist ein Ausführungsbeispiel eines Zwischenteils (oft auch als "Meniskusteil" bezeichnet) dargestellt, in Fig. 4 in einer perspektivischen Ansicht und in Fig. 5 in einer Aufsicht. Das Zwischenteil 2 weist eine erste, konkave Lagerfläche 20 auf, welche dem Femurteil 3 (siehe Fig. 6, Fig. 7) zugewandt ist. Die konkave Lagerfläche ist vorzugsweise sphärisch ausgestaltet und weist den gleichen Krümmungsradius auf wie die Lauffläche 30 des Femurteils 3, sodass sich maximale Kongruenz und damit eine möglichst geringe Flächenpressung ergibt. Weiterhin umfasst das Zwischenteil 2 eine zweite Lagerfläche 22 auf, welche mit der Gleitfläche 11 des Tibiateils 1 zusammenwirkt.

Man erkennt ferner, dass das Zwischenteil 2 eine bogenförmig, insbesondere kreissegmentförmig, gekrümmte Seitenfläche 21 aufweist. Die Seitenfläche 21 ist bei dem beschriebenen Ausführungsbeispiel des Zwischenteils 2 konkav ausgebildet. Der Krümmungsradius R2 dieser konkav ausgebildeten Seitenfläche 21 entspricht dem Krümmungsradius R1 der konvex ausgebildeten Begrenzungsfläche 11 (siehe Fig. 1) des Tibiateils 1.

Ferner erkennt man noch, dass sich die Seitenfläche 21 über einen bestimmten Bogenwinkel α erstreckt. Dieser Bogenwinkel α liegt vorzugsweise in einem Bereich von 25° bis 45°. Dadurch wird einerseits eine ausreichend grosse Seitenfläche 21 gewährleistet, die Kräfte in medialer/lateraler Richtung auf die Begrenzungsfläche 11 und damit auf das Tibiateil 1 und somit letztlich auf die Tibia übertragen kann, und andererseits wird auch eine ausreichend grosse Fläche zur Führung der Seitenfläche 21 des Zwischenteils 2 an der Begrenzungsfläche 11 entlang gewährleistet.

Der Krümmungsradius R1 der konvexen seitlichen Begrenzungsfläche 11 des Tibiateils 1 und dementsprechend auch der Krümmungsradius der konkaven Seitenfläche 21 des Zwischenteils 2 liegt vorzugsweise in einem Bereich von 30-50 mm. Dies entspricht im wesentlichen den Verhältnissen, wie sie beim natürlichen Knie vorkommen bzw. den Radien, mit welchen die Bewegung des femoro-tibialen Kontaktpunkts beim natürlichen Knie besonders gut approximiert wird.

Die Breite B des Tibiateils 1 liegt vorzugsweise im Bereich von 15-55 mm, die Länge L des Tibiateils liegt vorzugsweise im Bereich von 35-60 mm. Damit werden im wesentlichen diejenigen Fälle von Abmessungen abgedeckt, die bei der Tibia im Bereich des Knies beim Menschen vorkommen.

Die Materialien für das Tibiateil 1 und das Zwischenteil 2 sind übliche Materialien, die bereits für derartige Teile in der Anwendung sind. So ist das Tibiateil 1 typischerweise metallisch und kann z.B. aus einer Kobalt-Chrom-Legierung hergestellt sein. Für den Fall, dass eine zementfreie Implantation des Tibiateils 1 erfolgen soll, kann die der Tibia zugewandte Unterseite des Tibiateils mit einer entsprechenden Materialschicht versehen sein (z.B. mit einer Titanschicht). Das Zwischenteil 2 ist typischerweise aus einem Kunststoff wie Polyethylen, insbesondere aus ultrahochmolekularem Polyethylen, hergestellt, welches besonders gute Gleit- und Verschleisseigenschaften aufweist.

In Fig. 6 und Fig. 7 ist ein Ausführungsbeispiel eines Femurteils 3 der erfindungsgemässen monokondylären Kniegelenkprothese dargestellt. Man erkennt einen Verankerungszapfen 31, welcher nach der Präparation des Femurs in den Femur eingetrieben wird. Die Lauffläche 30 des Femurteils 3 ist vorzugsweise sphärisch ausgebildet und weist einen Krümmungsradius R_{K} auf, welcher dem Krümmungsradius der konkaven Lagerfläche 20 des Zwischenteils 2 entspricht, damit sich maximale Kongruenz und damit eine möglichst geringe Flächenpressung ergibt.

Hinsichtlich des Materials für das Femurteil 3 gilt, dass dieses ebenfalls aus einem für derartige Teile üblichen Material hergestellt ist, z.B. aus einer Kobalt-Chrom-Legierung. Sofern eine zementfreie Implantation erfolgen soll, können diejenigen Teile des Femurteils 3, die mit dem Femur in Kontakt kommen (z.B. der Verankerungszapfen 31 sowie die Rückfläche des Femurteils), mit einer entsprechenden Schicht (z.B. einer Titanschicht) versehen sein.

## Patentansprüche

1. Monokondyläre Kniegelenkprothese, mit einem Femurteil (3), welches eine konvexe Lauffläche (30) aufweist, ferner mit einem Tibiateil (1) mit einer dem Femurteil zugewandten Gleitfläche (10), und mit einem Zwischenteil (2), welches bei der Implantation zwischen das Femurteil (3) und das Tibiateil eingebracht wird und welches Zwischenteil (2) zwei Lagerflächen aufweist, nämlich eine erste, konkave Lagerfläche (20) zum Zusammenwirken mit der konvexen Lauffläche (30) des Femurteils (3), sowie eine zweite Lagerfläche (22) zum Zusammenwirken mit der Gleitfläche (10) des Tibiateils (1), wobei am Tibiateil (1) eine seitliche Begrenzungsfläche (11) vorgesehen ist, welche die Gleitfläche (10) des Tibiateils (1) seitlich begrenzt und in Richtung auf das Femurteil weisend vorsteht, um mit einer Seitenfläche (21) des Zwischenteils (2) zusammenwirken zu können, **dadurch gekennzeichnet, dass** die am Tibiateil (1) vorgesehene seitliche Begrenzungsfläche (11) bogenförmig gekrümmt ausgebildet ist, und dass das Zwischenteil (2) eine Seitenfläche (21) aufweist, welche entsprechend der am Tibiateil vorgesehenen, bogenförmig gekrümmten seitlichen Begrenzungsfläche gekrümmt ist.

2. Kniegelenkprothese nach Anspruch 1, bei welcher die am Tibiateil (1) vorgesehene seitliche Begrenzungsfläche kreissegmentförmig (R1) gekrümmt ausgebildet ist, und bei welcher die Seitenfläche des Zwischenteils (2) entsprechend kreissegmentförmig (R2) gekrümmt ausgebildet ist.

3. Kniegelenkprothese nach Anspruch 1 oder 2, bei welcher die am Tibiateil (1) vorgesehene seitliche Begrenzungsfläche (11) konvex und die entsprechende Seitenfläche (21) des Zwischenteils (2) konkav ausgebildet ist.

4. Kniegelenkprothese nach einem der vorangehenden Ansprüche, bei welcher der Krümmungsradius (R1) der seitlichen Begrenzungsfläche (11) des Tibiateils (1) und dementsprechend der Krümmungsradius (R2) der Seitenfläche (21) des Zwischenteils (2) im Bereich von 30-50 mm liegt.

5. Kniegelenkprothese nach einem der vorangehenden Ansprüche, bei welcher die Breite (B) des Tibiateils (1) im Bereich von 15-55 mm liegt, und bei welchem die Länge (L) des Tibiateils (1) im Bereich von 35-60 mm liegt.

6. Kniegelenkprothese nach einem der vorangehenden Ansprüche, bei welcher die bogenförmige Seitenfläche (21) des Zwischenteils (2) sich über einen Bogenwinkel (α) erstreckt, der im Bereich von 25°-45° liegt.

## Claims

1. Mono-condylar knee joint prosthesis including a femur part (3) having a convex running surface (30), further including a tibia part (1) having a sliding surface (10) which faces the femur part and including an intermediate part (2) which is introduced between the femur part (3) and the tibia part during the implantation, and said intermediate part (2) having two bearing surfaces, namely a first, concave bearing surface (20) for cooperating with the convex running surface (30) of the femur part (3) as well as a second bearing surface (22) for cooperating with the sliding surface (10) of the tibia part (1), with a lateral boundary surface (11) being provided at the tibia part (1) which laterally bounds the sliding surface (10) of the tibia part (1) and projects pointing in the direction towards the femur part in order to be able to cooperate with a side surface (21) of the intermediate part (2), **characterized in that** the lateral boundary surface (11) which is provided at the tibia part (1) is curved in the shape of an arch; and **in that** the intermediate part (2) has a side surface (21) which is curved corresponding to the lateral boundary surface which is provided at the tibia part and which is curved in the shape of an arch.

2. Knee joint prosthesis in accordance with claim 1, in which the lateral boundary surface which is provided at the tibia part (1) is formed to be curved in the shape of a circular segment (R1) and in which the side surface of the intermediate part (2) is formed to be correspondingly curved in the shape of a circular segment (R2).

3. Knee joint prosthesis in accordance with claim 1 or claim 2, in which the lateral boundary surface (11) which is provided at the tibia part (1) is convex and the corresponding side surface (21) of the intermediate part (2) is formed to be concave.

4. Knee joint prosthesis in accordance with any one of the preceding claims, in which the radius of curvature (R1) of the lateral boundary surface (11) of the tibia part (1), and accordingly the radius of curvature (R2) of the side surface (21) of the intermediate part (2), lies in the range from 30 - 50 mm.

5. Knee joint prosthesis in accordance with any one of the preceding claims, in which the width (B) of the tibia part (1) lies in the range from 15 - 55 mm and in which the length (L) of the tibia part (1) lies in the range from 35 - 60 mm.

6. Knee joint prosthesis in accordance with any one of the preceding claims, in which the arcuate side surface (21) of the intermediate part (2) extends over an angle of arc (α) which lies in the range from 25° - 45°.

## Revendications

1. Prothèse du genou à un seul compartiment, comprenant une partie fémorale (3) dotée d'une surface de contact convexe (30), ainsi qu'une partie tibiale (1) munie d'une surface de glissement (10) tournée vers la partie fémorale, et une partie intercalaire (2) qui est insérée entre la partie fémorale (3) et la partie tibiale au stade de l'implantation, laquelle partie intercalaire (2) comporte deux surfaces d'appui, c'est-à-dire une première surface d'appui concave (20) conçue pour coopérer avec la surface de contact convexe (30) de la partie fémorale (3), ainsi qu'une seconde surface d'appui (22) conçue pour coopérer avec la surface de glissement (10) de la partie tibiale (1), sachant qu'une surface latérale de délimitation (11), prévue sur la partie tibiale (1), délimite latéralement la surface de glissement (10) de ladite partie tibiale (1) et fait saillie en direction de la partie fémorale, pour pouvoir coopérer avec une surface latérale (21) de la partie intercalaire (2), **caractérisée par le fait que** la surface latérale de délimitation (11), prévue sur la partie tibiale (1), est réalisée avec courbure en arc, et **par le fait que** la partie intercalaire (2) présente une surface latérale (21) offrant une courbure correspondant à la surface latérale de délimitation à courbure en arc, prévue sur la partie tibiale.

2. Prothèse du genou selon la revendication 1, dans laquelle la surface latérale de délimitation prévue sur la partie tibiale (1) est réalisée avec courbure en forme de segment circulaire (R1), et dans laquelle la surface latérale de la partie intercalaire (2) est réalisée, de façon correspondante, avec courbure en forme de segment circulaire (R2).

3. Prothèse du genou selon la revendication 1 ou 2, dans laquelle la surface latérale de délimitation (11) prévue sur la partie tibiale (1) est de réalisation convexe, et la surface latérale correspondante (21) de la partie intercalaire (2) est de réalisation concave.

4. Prothèse du genou selon l'une des revendications précédentes, dans laquelle le rayon de courbure (R1) de la surface latérale de délimitation (11) de la partie tibiale (1) et, de façon correspondante, le rayon de courbure (R2) de la surface latérale (21) de la partie intercalaire (2), se situent dans la plage de 30-50 mm.

5. Prothèse du genou selon l'une des revendications précédentes, dans laquelle la largeur (B) de la partie tibiale (1) se situe dans la plage de 15-55 mm, et dans laquelle la longueur (L) de ladite partie tibiale (1) se situe dans la plage de 35-60 mm.

6. Prothèse du genou selon l'une des revendications précédentes, dans laquelle la surface latérale (21) en arc de la partie intercalaire (2) s'étend sur un angle d'arc (α) situé dans la plage de 25°-45°.
